(19) Europäisches Patentamt
European Patent Office
Office européen des brevets

(11) Publication number: **0 224 596**
**B1**

# (12) EUROPEAN PATENT SPECIFICATION

(45) Date of publication of patent specification: **28.11.90**

(51) Int. Cl.$^5$: **A 61 K 31/09,** A 61 K 31/36 // A61K35/78

(21) Application number: **86903582.4**

(22) Date of filing: **05.06.86**

(86) International application number: **PCT/JP86/00281**

(87) International publication number: **WO 86/07256 18.12.86 Gazette 86/27**

## (54) Use of dibenzocyclooctadiene lignans extracted from Schisandrae Fructus for the manufacture of a medicament for the treatment of hepatic failure.

(30) Priority: **07.06.85 JP 122560/85**

(43) Date of publication of application: **10.06.87 Bulletin 87/24**

(45) Publication of the grant of the patent: **28.11.90 Bulletin 90/48**

(84) Designated Contracting States: **BE FR IT**

(56) References cited:
**JP-A-5 432 633**
**JP-A-5 522 612**

**CHEMICAL ABSTRACTS, vol. 99, no.6, Abstract no. 99: 43608r (08.08.83) Columbus, Ohio,**

**CHEMICAL ABSTRACTS, vol. 97, no.19, Abstract no. 97:157710g (08.11.82) (Columbus, Ohio, US)**

**CHEMICAL ABSTRACTS, vol. 97, no. 17, Abstract no. 97: 138610h (25.10.82) (Columbus, Ohio, US)**

(73) Proprietor: **TSUMURA JUNTENDO, INC.**
**4-10, Nihonbashi 3-chome**
**Chuou-ku, Tokyo 103 (JP)**

(72) Inventor: **ABURADA, Masaki 10-4-4, Kohokudai**
**Abiko-shi**
**Chiba 270-11 (JP)**
Inventor: **TAKEDA, Shigefumi 3-8-10, Matsuba**
**Ryugasaki-shi**
**Ibaraki 301 (JP)**
Inventor: **TAGUCHI, Heihachirou 2-40-8,**
**Shimoishihara**
**Choufu-shi**
**Tokyo 182 (JP)**
Inventor: **IKEYA, Yukinobu 391, Sakae-machi**
**6-chome**
**Ushiku-shi**
**Ibaraki 300-12 (JP)**

(74) Representative: **Smulders, Theodorus A.H.J., Ir. et al**
**Vereenigde Octrooibureaux Nieuwe Parklaan 107**
**NL-2587 BP 's-Gravenhage (NL)**

**EP 0 224 596 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
CHEMICAL ABSTRACTS, vol. 97, no. 5, Abstract no. 97: 34258h (02.08.82) (Columbus, Ohio, US)

CHEMICAL ABSTRACTS, vol. 97, no. 3, Abstract no. 97: 16547d (19.07.82) (Columbus, Ohio, US)

CHEMICAL ABSTRACTS, vol. 96, no. 19, Abstract no. 96: 155070d (10.05.82) (Columbus, Ohio, US)

CHEMICAL ABSTRACTS, vol. 92, no. 5, Abstract no. 92: 34283j (04.02.80) (Columbus, Ohio, US)

CHEMICAL ABSTRACTS, vol. 90, no. 11, Abstract no. 90: 83582y (12.03.79) (Columbus, Ohio, US)

CHEMICAL ABSTRACTS, vol. 88, no. 8, Abstract no. 88: 55007e (20.02.78) (Columbus, Ohio, US)

CHEMICAL ABSTRACTS, vol. 102, no. 23, Abstract no. 102: 197529h (10.06.85) (Columbus, Ohio, US), Planta Med., 50(3), 213

CHEMICAL ABSTRACTS, vol. 102, no. 25, Abstract no. 102: 214941f (24.06.85) (Columbus, Ohio, US), Nippon Yakurigaku Zasshi, 85(3), 193

CHEMICAL ABSTRACTS, vol. 103, no. 15, Abstract no. 103: 116249n (14.10.85) (Columbus, Ohio, US), Jpn. J. Pharmacol., 38(4), 347

CHEMICAL ABSTRACTS, vol. 103, no. 25, Abstract no. 103: 206499y (23.12.85) (Columbus, Ohio, US)

CHEMICAL ABSTRACTS, vol. 104, no. 17, Abstract no. 104: 142225s (28.04.86) (Columbus, Ohio, US), Nippon Yakurigaku Zasshi, 87 (2), 169

CHEMICAL ABSTRACTS, vol. 104, no. 19, Abstract no. 104: 161941p (12.05.86) (Columbus, Ohio, US); Adv. Chin. Med. Mater. Res., Int. Symp., Meeting Date 1984, 257-67. Edited by: Chang, H.M. World Sci.: Singapore, Singapore

**Description**

The present invention pertains to a therapeutic drug for hepatic failure, and, more particularly, to such a drug consisting of dibenzocyclooctadiene lignans extracted from Schisandrae Fructus.

Hepatic failure caused by widespread necrosis of liver cells is a disease which presents symptoms such as liver atrophy, progressive jaundice, esophago hemorrhage and digestive system hemorrhage. In the prior art, hepatic failure patients have been undergoing only assisted treatments such as tentative maintenance and compensation of liver function. However, these conventional treatments are no more than symptomatic treatment, and no therapeutic drugs effective for curing hepatic failure have been previously known in the art. Consequently a drug effective for curing hepatic failure has long been desired.

Therefore the object of the present invention is the use of dibenzocyclooctadiene lignans extracted from Schisandrae Fructus, represented by the following general structural formula:

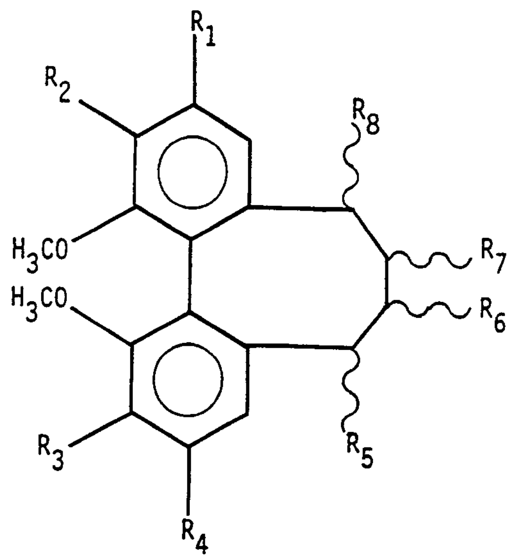

wherein both $R_1$ and $R_2$ are $-OCH_3$, or $R_1$ together with $R_2$ is $-OCH_2O-$;

both $R_3$ and $R_4$ are $-OCH_3$, or $R_3$ together with $R_4$ is $-OCH_2O-$;

$R_5$ is ◀ H, . . . $CH_3$ or ◀ OH, using the convention where dotted line indicates a bond down from the plane of the paper, the triangles indicate bonds up from such plane, and wavy lines indicates both possibilities; and

$R_6$, $R_7$ and $R_8$ are ◀ H or . . . $CH_3$, for the manufacture of a therapeutic drug for the treatment of hepatic failure.

Best Mode for Carrying Out the Invention

Deoxyschizandrin

Korean Gomisi (Schizandra chinensis Baill.) used to obtain a therapeutic drug for hepatic failure according to the present invention is a deciduous tree belonging to the Magnoliaceae family which grows indigenously in Japan, Korea and China. Dried mature fruit of Schizandra chinensis Baill. is a drug called Gomisi (Schisandrae Fructus) or Kita-Gomisi. The Gomisi or Kita-Gomisi is commercially available.

A compound where $R_1$, $R_2$, $R_3$ and $R_4$ are $-OCH_3$, $R_5$ is ◀ H, $R_6$ is . . . $CH_3$, $R_7$ is ◀ H and $R_8$ is . . . $CH_3$ in the above general formula is called deoxyschizandrin. Deoxyschizandrin may be obtained by the following processes.

Schisandrae Fructus is extracted with an organic solvent such as petroleum ether, ether, ethanol and methanol. This extract, or its concentrate obtained by removing the organic solvent by evaporation is subjected to silica gel column chromatography and eluted with one or more solvents selected from benzene, n-hexane, acetone, ethyl acetate, methanol and ethanol to obtain an eluate. The solvent is removed from the eluate to obtain a crude fraction containing deoxyschizandrin. The crude fraction is subjected to preparative thin layer chromatography (PTLC), and developed with one or more solvents selected from benzene, ether, n-hexane, acetone, ethyl acetate, methanol and ethanol. The zone containing deoxyschizandrin whose presence can be identified by UV irradiation is collected and extracted with single or mixture of high polarity solvents such as chloroform, methanol and the like. The extract gives deoxyschizandrin by the removal of solvents. Repeated PTLC of deoxyshizandrin obtained by the above procedure give pure deoxyshizandrin. Pure deoxyschizandrin can be obtained by recrystallized from one or more solvents selected from ether, chloroform, n-hexane, acetone, methanol, ethanol. A more detailed explanation regarding preparation of deoxyschizandrin is provided with the following Production Example

1. The percentages and ratios referred to in this specification are expressed by volume/volume unless otherwise expressly noted.

Production Example 1

The dried powders (9.34 kilograms) of Schisandra chinensis Baill. were extracted with petroleum ether (24 liters) under reflux (36°C, 6 hours). And the extract was filtered to remove a residue. The residue was further extracted twice in the same manner. The three extracts were combined and concentrated by removing petroleum ether under reduced pressure to obtain dried extractive (1.15 kilograms).

The dried extract (1.15 kilograms) was subjected to silica gel column chromatography (Kieselgel 60, 70—230 mesh Merck) developing with benzene containing increasing amounts of acetone. The fraction (5 liters) eluted with benzene-acetone (92:8) was concentrated by removing the solvent to give the crude fractions of deoxyshisandrin (129.8 grams).

The above crude fraction dried extractive (25.4 grams) was subjected to PTLC (thin layer plate:Kieselgel $PF_{254}$ (Merck); developing solvent:benzene-ether 5:1)). After developing, the zone of RF0.71 showing UV 254 nm absorption was extracted with chloroform-methanol (4:1)). The extract was concentrated by removing the solvent to give a residue. This residue was further purified by PTLC (thin layer plate:Kieselgel $PF_{254}$ (Merck); developing solvent: hexan-ethyl acetate (4:1)). After developing, the zone of Rf0.44 showing UV (254 nm) absorption was extracted with chloroform-methanol (4:1). The extract was concentrated by removing solvent to give a residue. The residue was recrystallized from the mixture of n-hexane and ether to give colorless crystal. The physical and chemical properties of this crystal were identical with those of deoxyschizandrin described in the literature (Chem. Pharm. Bull, 27(11)2695—2709 (1979)).

Gomisin N

A compound where $R_1$ together with $R_2$ is $—OCH_2O—$, $R_3$ and $R_4$ are $—OCH_3$, $R_5$ is . . . $CH_3$, $R_6$ is ◀ H, $R_7$ is . . . $CH_3$ and $R_8$ in the above general formula is ◀ H is called gomisin N. Gomisin N may be obtained by extracting of Schisandrae Fructus with lower hydrocarbons to give an extract, removing solvents and volatile components from the extract to give a residue, and subjecting the residue to chromatography.

Pulverized powder of Schisandrae Fructus is extracted into lower hydrocarbon such as petroleum ether, n-hexane, benzene and the like at a temperature ranging from 30°C to the boiling point, preferably under reflux. The extract obtained by extracting plural times are combined and concentrated by removing the lower hydrocarbon to give a dried extract. This extract is steam-distilled to remove volatile components such as essential oil.

The residue (essential oil free portion soluble in lower hydrocarbons) is subjected to column chromatography using adsorbent such as silica gel, alumina, florisil, polyamide and celite. The fractions eluted with an organic solvent such as n-hexane, benzene, chloroform, acetone, ether, ethyl acetate, ethanol and methanol or mixture of these solvents, is concentrated by removing solvents to give gomisin N.

Gomisin N may be obtained also by extracting from Schisandrae Fructus with lower alcohol such as methanol and ethanol. In this case, the extract with lower alcohol is concentrated to give an extract. This extract is dissolved in water, adsorbed on the adsorbent such as celite and cellulose powder and eluted with n-hexane, petroleum ether, benzene, chloroform and ether. The eluent is concentrated and subjected to the above chromatography to give gomisin N. A more detailed explanation regarding preparation of gomisin N is provided with the following Production Example 2.

Production Example 2

Pulverized Schisandrae Fructus (4.67 kilograms) was mixed into petroleum ether (12 liters, boiling point: 37—39°C), and extracted under reflux for 8 hours. This extracting process was repeated three times. These three extracts were combined and concentrated by removing ether under reduced pressure to give an extract (546 grams). This extract was suspended in water (1200 ml), and essential oil was removed by steam distilling for 3 hours to giver a residue. The undistilled part was extracted from the residue with ether six times and the ethereal extracts were combined, and concentrated by removing ether to obtain an essential oil free portion (491 grams) which is soluble in petroleum ether.

The above essential oil free portion (491 grams) was dissolved in n-hexane (1 liter), injected to a column in which silica gel (5 kilograms) swollen with n-hexane had previously been packed in a glass tube (diameter: 12 cm; length: 110 cm), and developed with first n-hexane, then benzene and finally benzene-acetone mixture. The two parts eluted respectively with benzene-acetone (47:3) and benzene-acetone (46:4) were combined, and concentrated by removing the solvent under reduced pressure to give a residue. The residue was dissolved in n-hexane-ether mixture at elevated temperature, left at room temperature to crystallize, and filtered to obtain crystal of gomisin N (14 grams, 0.3%).

Schizandrin. Gomisin A

Among the compounds represented by the above general formula, a compound where $R_1$, $R_2$, $R_3$ and $R_4$ are $—OCH_3$, $R_5$ is ◀ OH, $R_6$ is . . . $CH_3$, $R_7$ is ◀ H and $R_8$ is . . . $CH_3$ is called schizandrin. Whereas a compound where $R_1$ together with $R_2$ is $—OCH_2O—$, $R_3$ and $R_4$ are $—OCH_3$, $R_5$ is ◀ OH, $R_6$ is . . . $CH_3$, $R_7$ is ◀ H and $R_8$ is . . . $CH_3$ is called gomisin A.

Schizandrin may be obtained by the method described in the literature (N. K. Kochetkov, etc.: Zh. Obshch. Khim., volume 31, page 3454, 1961). Specifically, Schizandrin may be obtained by the following processes. An extract obtained by extracting from Schisandrae Fructus with ligroin is treated with methanol. After the methanol-soluble part is hydrolyzed with NaOH (1N, 85% methanol solution), the neutral part is subjected to chromatography with alumina, developed with ligroin, benzene and chloroform, and the eluate with chloroform gives Schizandrin (0.14%).

Gomisin A may be obtained by the method described in the literature (Taguchi, et al.: Chem. Pharm. Bull. (Tokyo), volume 23, page 3296, 1975; Taguchi, et al.: Chem. Pharm. Bull (Tokyo), volume 25, page 364, 1977). Specifically, gomisin A may be obtained by the following processes. The extract of Schisandrae Fructus with hot lower hydrocarbons (such as petroleum ether, n-hexane and benzene) is concentrated by removing the solvent to give a residue. The residue is dissolved in water, and steam-distilled to remove the essential oil. The undistilled part is subjected to chromatography with silica gel, and developed with n-hexane, benzene or acetone or these mixture to separate gomisin A. This method may also give schizandrin. A more detailed explanation regarding preparation of schizandrin and gomisin A is provided with the following Production Example 3.

Production Example 3

Pulverized Schisandrae Fructus (1.38 kilograms) was extracted with petroleum ether (3 liters) under reflux four times (8 hours each). The obtained extracts were combined and concentrated under reduced pressure to obtain the petroleum ethereal extract (188 grams). This extract was suspended in water (450 ml), and steam-distilled for 3 hours to remove the essential oil. The undistilled part was extracted with ether four times and the ethereal extracts were combined and concentrated by removing the ether to give an essential oil free part (179 grams, fraction A) which is soluble in petroleum ether.

Schisandrae Fructus after extracting with petroleum ether was further extracted with hot methanol three times (8 hours each), then the methanolic extracts were combined and concentrated to give a dried methanolic extract (383 grams). The methanolic extract was dissolved in water (580 ml) and extracted with ethyl acetate (850 ml) three times. Three eluates with ethyl acetate were combined and concentrated at reduced pressure to give an extract (78 grams). This extract was dissolved in methanol, and mixed with celite (No. 535, Johns-Manville, 300 grams). The mixture was chromatographed, developing with n-hexane (2 liters) and the eluate was concentrated at reduced pressure to obtain an extract (20.8 grams, fraction B).

Fraction A (179 grams) and Fraction B (20.8 grams) were combined, subjected to chromatography with silica gel (1200 grams), developing with first n-hexane, then benzene and finally benzene-aceton solvent systems. The eluate with benzene-acetone (4:1) and the eluate with benzene-acetone (3:1) were combined and concentrated to give a residue. This residue was rechromatographed on silica gel (480 grams) and developing with benzene-ether solvent systems. The eluate with benzene-ether (4:1) was crystallized from methanol to obtain gomisin A (3.04 grams, 0.22%).

The eluate obtained with benzene-acetone (7:3) by the above chromatography and the eluate obtained with benzene-acetone (3:2) were combined and concentrated to give a residue (8.3 grams). This residue was rechromatographed on silica gel (180 grams) developing with n-hexane-acetone solvent systems. The eluate with n-hexane-acetone (22:3) was crystallized from n-hexane-ether to obtain schizandrin (3.5 grams, 0.25%).

γ-Shizandrin

Among the compounds represented by the above general formula, a compound where $R_1$ together with $R_2$ is $—OCH_2O—$, $R_3$ and $R_4$ are $—OCH_3$, $R_5$ is ◄ H, $R_6$ is . . . $CH_3$, $R_7$ is ◄ H and $R_8$ is . . . $CH_3$ is called γ-schizandrin. The γ-schizandrin may be obtained by the following processes. Schisandrae Fructus, that is the dried fruits of Schizandra chinensis Baill., is extracted with an organic solvent such as petroleum ether, ether, n-hexane, ethanol and methanol. The extract, as is, or its concentrate by removing the organic solvent is subjected to column chromatography with silica gel, eluted with one or more solvent(s) selected from petroleum ether, n-hexane, benzene, acetone, ethyl acetate, methanol and ethanol to give an eluate. The eluate is concentrated by removing the solvents to give the crude fraction of γ-schizandrin. This crude fraction is recrystallized from the mixture of n-hexane and ether, and further recrystallized repeatedly from the mixture of n-hexane and ether, or methanol, to obtain γ-schizandrin. A more detailed explanation regarding preparation of γ-schizandrin is provided with the following Production Example 4.

Production Example 4

Dried powder (9.34 kilograms) of Schizandra chinensis Baill. was extracted with petroleum ether (24 liters) under reflux (36°C, 6 hours). The extract, after cooling, was filtered to give a residue. The residue was further extracted twice in the same manner. The total three extracts were combined and concentrated by removing petroleum ether under reduced pressure to obtain a dried extract (1.15 kilograms).

This dried extract (1.15 kilograms) was subjected to silica gel column chromatography (Kieselgel 60, 70—230 mesh, Merck, 10 kilograms), and eluted with benzene containing increasing amounts of acetone. The eluate with benzene-acetone (92:8, 5 liters) was concentrated by removing the solvent to give a dried extract (129.8 grams). This dried extract (129.8 grams) was recrystallized from the mixture of n-hexane and ether, to give colorless crystal (15.44 grams). This colorless crystal (15.44 grams) was further recrystallized

three times from methanol, to obtain final colorless prism crystal (1.102 grams, 0.012%). The physical and chemical properties of the crystal were identical with those of γ-schizandrin described in the literature (Chem. Pharm. Bull., 30(1), 132—139 (1982)).

Wuweizisu C

Among the compounds represented by the above general formula, a compound where $R_1$ together with $R_2$ is $—OCH_2O—$, $R_3$ together with $R_4$ is $—OCH_2O—$, $R_5$ is . . . $CH_3$, $R_6$ is ◀ H, $R_7$ is .∵. $CH_3$ and $R_8$ is ◀ H is called Wuweizisu C. Wuweizisu C may be obtained by the following processes. Schisandrae Fructus, that is dried fruits of Schizandra chinensis Baill., is extracted with an organic solvent such as petroleum ether, ether, n-hexane, ethanol and methanol. The eluate, as is, or its concentrate by removing the organic solvent is subjected to silica gel column chromatography, and eluted with one or more solvent(s) selected from petroleum ether, n-hexane, benzene, acetone, ethyl acetate, methanol and ethanol, to give an eluate. The eluate is concentrated by removing the solvent to give the crude fraction of wuweizisu C. This crude fraction is subjected to silica gel column chromatography, eluted with one or more solvent(s) selected from n-hexane, petroleum ether, benzene, ether, acetone, ethyl acetate, methanol and ethanol, and concentrated by removing the solvent to give final crude fraction of wuweizisu C. The final crude fraction of fraction is recrystallized from the mixture of n-hexane and ether, or methanol, to obtain wuweizisu C. A more detailed explanation regarding preparation of wuweizisu C is provided with the following Production Example 5.

Production Example 5

Dried powder (4.67 kilograms) of Schizandra chinensis Baill. was extracted with petroleum ether under reflux (36°C, 6 hours). The extractive, after cooling, was filtered to give a residue. The residue was further reextracted twice in the same manner. The total three extracts were combined and concentrated by removing petroleum ether under reduced pressure to obtain a dried extract (546 grams).

This dried extract (546 grams) was subjected to silica gel column chromatography (Kieselgel 60, 70—230 mesh, Merck, 10 kilograms), and eluted with first n-hexane-benzene (1:1, 4 liters), then n-hexane-benzene (9:1, 3 liters), then benzene (6.5 liters) and finally benzene containing increasing amounts of acetone. The eluate (6.5 liters) with benzene, the eluate (5 liters) with benzene-acetone (98:2) and the eluate (5 liters) with benzene-acetone (96:4; were combined and concentrated by removing the solvent to give a dried extract (114.0 grams). This extract (114 grams) is subjected to silica gel column chromatography (Kieselgel 60, 70—230 mesh, Merck, 2 kilograms), and eluted with n-hexane containing increasing amounts of ethylacetate. The eluate (2 liters) with n-hexane-ethylacetate (92:8) was concentrated by removing the solvent to give a residue (6.73 grams). This residue (6.73 grams) was recrystallized from methanol to obtain colorless crystal (3.346 grams, 0.072%). The physical and chemical properties of this crystal were identical with those of wuweizisu C described in the literature (Chem. Pharm. Bull., 30(9), 3207—3211 (1982)).

Pharmacological Assessment

The effectiveness of these therapeutic drugs for hepatic failure is demonstrated by the following Experiments.

Experiment 1

(Effect on survival ratio of mice with acute hepatic failure)

Seven groups of male babl/c strain mice (six weeks of age) were used. Each group included 40 mice. Acute hepatic failure was induced by the injection of 1 µg/mouse of LPS (lipopolysaccharide, Difco Laboratories) to mice that had been injected with 1 mg/mouse of heat-killed P. acnes (Propionibacterium acnes, First Department of Biochemistry in Osaka City Univ. Medical School) seven days before. The above obtained compounds (deoxyschizandrin, gomisin N, schizandrin, gomisin A, γ-shizandrin and wuweizisu C) which had been suspended in 0.5% CMC (carboxymethyl cellulose, Wako Junyaku Kogyo, Co. Ltd.), was given p.o. to each group eight hours after the injection of LPS. Only vehicle (CMC) was given p.o. to one remaining group as a control group.

Survival ratio of mice was periodically examined twenty-four hours after induction of acute hepatic failure. Each dose of the above compounds and corresponding survival ratio twenty-four hours after the injection of LPS are shown in Table 1.

TABLE 1
Effect on acute hepatic failure

| Compound | Dose (mg/kg) | Survival Ratio (%) |
|---|---|---|
| Control | — | 7.5 |
| Deoxyschizandrin | 50 | 55 |
| Gomisin N | 20 | 80 |
| Schizandrin | 50 | 50 |
| Gomisin A | 10 | 80 |
| γ-Schizandrin | 50 | 60 |
| Wuweizisu C | 20 | 70 |

Experiment 2
(Effect on serum GOT (glutamic oxaloacetic transaminase) of mice with acute hepatic failure)

Three groups of male Wistar rats were used. Each group included 45 rats. Each rat in all groups was injected with 10 mg/rat of P. acnes. Four days after, gomisin A (100 mg/kg, 50 mg/kg) obtained in Production Example 3 were suspended in 2% Tween® 80/saline and administered i.p. to respectively two of three groups. Only vehicle (2% Tween 80®/saline) was administered i.p. to one remaining group as a control group. All rats were injected with 50 μg/rat of LPS, seven days after the injection of P. acnes.

Twenty-four hours after the injection of LPS, blood samples were collected from surviving rats by heart puncture, and serum GOT activities were measured. GOT is one of amino acid transferases contained in liver cells. High serum GOT activities reflect large number of destoyed liver cells.

As a result, while the survival ratio of the control group was 8.8%, the survival ratios of two gomisin A (100 mg/kg, 50 mg/kg) administered groups were respectively 100%, 83.3%.

While average serum GOT activities of surviving rats in the control group was 7000 IU, average serum GOT activities of gomisin A (100 mg/kg) administered group was reduced to 140 IU.

These results indicate the effectiveness of these therapeutic drugs for hepatic failure.

$LD_{50}$ of the compounds obtained in Production Examples 1—5 in ddY mice are shown in Table 2. And estimated clinical daily doses of the present therapeutic drugs for patients with hepatic failure are also given in Table 2.

TABLE 2
$LD_{50}$ of the present compounds administered p.o. in mice and their estimated clinical daily doses in patients

| Compound | $LD_{50}$ (mg/kg) | Clinical daily dose (mg/kg) p.o. | i.v. |
|---|---|---|---|
| Deoxyshizandrin | 2000 | 45—100 | 7.5—10 |
| Gomisin N | 2000 | 30—60 | 3—6 |
| Schizandrin | 1448 | 45—100 | 15—30 |
| Gomisin A | 777 | 15—30 | 5—10 |
| γ-Schizandrin | 2000 | 45—100 | 2.5—5 |
| Wuweizisu C | 2000 | 30—60 | 2.5—5 |

Although only seven examples of dibenzocyclooctadiene lignans represented by general formula were explained with Examples above, all compounds included in the general formula described in Claims are believed to have similar effects, provided that the subtitutional conditions are satisfied.

Preparations

Medicines in the wide variety forms such as liquid, powder, granulated powder, pill, tablet, enteric-coated tablets and capsules may be produced in the conventional manner by using the drug compounds according to the present invention together with suitable solvents, excipients and adjuvants. The above medicines may be compounded with other medicinal active ingredient(s) in case of prescribing. For oral administration medicines in the form of liquid, powder, granulated powder, pill, tablet, enteric-coated tablet and capsule may be prescribed by using at least one excipient such as starch, lactose, sucrose, mannitol and carboxymethylcellulose.

The above medicines may also be produced by using brighteners such as magnesium stearate, sodium lauric acid and talc; binders such as dextrin, crystalline cellulose, polyvinyl pyrrolidone, gum arabic, corn starch and gelatin; and breaking agents such as potato starch and carboxymethylcellulose. The drug compounds according to the present invention may be administered as suspension, emulsion, syrup and elixir, which may contain one or more taste- and odor-correcting agents and coloring agents.

In case of production of injecting medicines diluents such as injecting distilled water, physiological salt solution, dextrose solution, an injecting vegetable oil, propylene glycol and polyethylene glycol may be used. Further, isotonic agents, stabilizers, antiseptics anodynes may be added if necessary or desirable. It is preferable to dissolve the injecting medicines in a sterilized injecting medium.

More detailed explanations regarding preparation of medicines using the compounds according to the present invention are provided with the following Prepartion Examples.

Preparation Example 1

Deoxyschizandrin (0.5 g) obtained by Production Example 1 was finely powdered and mixed with lactose (90.5 g) and magnesium stearate (9 g). The mixture was tabletted on tabletting machine to obtain tablets (diameter 20 mm; weight 2.3 g). The tablets were pulverized by an oscillator, graded and sieved to obtain granulated powder medicine (20—50 mesh).

The granulated powder medicine (1 g) contains 5 mg of deoxyschizandrin. Three doses are to be administered a day (each dose containing 3—6 g of the above medicine in accordance with condition).

Preparation Example 2

Gomisin N (1 g) obtained by Production Example 2 was finely powdered and mixed with lactose (90 g) and magnesium stearate (9 g). The mixture was tabletted on tabletting machine to obtain tablets (diameter 20 mm; weight 2.3 g). The tablets were pulverized by an oscillator, graded and sieved to obtain granulated powder medicine (20—50 mesh).

This granulated powder medicine (1 g) contains 10 mg of gomisin N. Three doses are to be administered a day (each dose containing 1—2 g of the above) medicine in accordance with condition).

Preparation Example 3

Gomisin A (0.5 g) obtained by Production Example 3 was finely powdered and mixed with lactose (90.5 g) and magnesium stearate (9 g). The mixture was tabletted on tabletting machine to obtain tablets (diameter 20 mm; weight 2.3 g). The tablets were pulverized by an oscillator, graded and sieved to obtain granulated powder medicine (20—50 mesh).

This granulated powder medicine (1 g) contains 5 mg of gomisin A. Three doses are to be administered a day (each dose containing 1—2 g of the above medicine in accordance with condition).

Preparation Example 4

Schizandrin (2.5 g) obtained by Production Example 3 was mixed with microcrystalline cellulose (90 g) and magnesium stearate (7.5 g). The mixture was tabletted on tabletting machine to obtain tablets (diameter 9 mm; weight 200 mg).

This tablet contains 5 mg of Schizandrin. Three doses (3—6 tablets each) are to be administered a day.

Preparation Example 5

γ-schizandrin (2.5 g) obtained by Production Example 4 was mixed with microcrystalline cellulose (90 g) and magnesium stearate (7.5 g). The mixture was tabletted on single punch machine to obtain tablets (diameter 9 mm; weight 200 mg).

This tablet contains 5 mg of γ-schizandrin. Three doses (3—6 tablets each) are to be administered a day.

Preparation Example 6

Wuweizisu C (2.5 g) obtained by Production Example 5 was mixed with microcrystalline cellulose (90 g) and magnesium stearate (7.5 g). The mixture was tabletted on single punch machine to obtain tablets (diameter 9 mm; weight 200 mg).

This tablet contains 5 mg of wuweizisu C. Three doses (3—6 tablets each) are to be administered a day.

Preparation Example 7

Gomisin N (10 g) obtained by Production Example 2 was finely powdered and mixed with lactose (90 g). Each 100 mg of the mixture was packed in a hard capsule to obtain capsule medicine.

This capsule contains 5 mg of gomisin N. Three doses (1—2 capsules each) are to be administered a day.

Preparation Example 8

Schizandrin (5 g) obtained by Production Example 3 was finely powdered and mixed with lactose (95 g). Each 100 mg of the mixture was packed in a hard capsule to obtain capsule medicine.

This capsule contains 5 mg of schizandrin. Three doses (3—6 capsules each) are to be administered a day.

Preparation Example 9

γ-Schizandrin (5 g) obtained by Production Example 4 was finely powdered and mixed with lactose (90 g). Each 100 mg of the mixture was packed in a hard capsule to obtain capsule medicine.

This capsule contains 5 mg of γ-schizandrin. Three doses (3—6 capsules each) are to be administered a day.

Preparation Example 10

Deoxyschizandrin (10 mg) obtained by Production Example 1 was packed and sealed within a sterilizing vial. For practical use this sealed drug is dissolved in 5% glucose solution or physiological salt solution, and injected by intravenous drip (500 ml/2—4 hours).

Preparation Example 11

Gomisin A (10 mg) obtained by Production Example 3 was packed and sealed within a sterilizing vial. For practical use this sealed drug is dissolved in 5% glucose solution or physiological salt solution, and injected by intravenous drip (500 ml/2—4 hours).

Preparation Example 12

Wuweizisu C (5 mg) obtained by Production Example 5 was packed and sealed within a sterilizing vial. For practical use this sealed drug is dissolved in 5% glucose solution or physiological salt solution, and injected by intravenous drip (500 ml/2—4 hours).

**Claims**

1. Use of a compound represented by the following general structural formula:

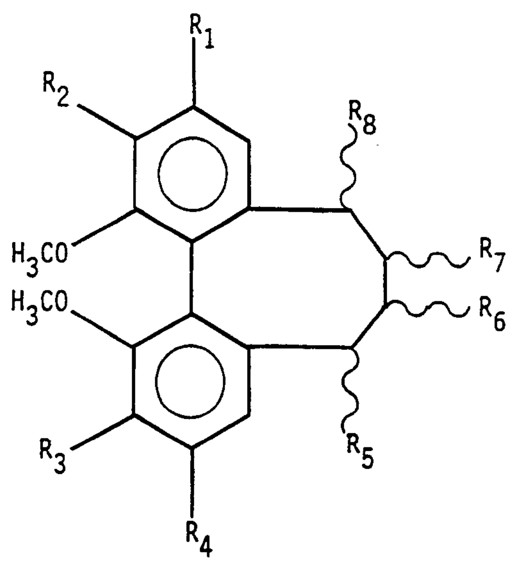

wherein both $R_1$ and $R_2$ are $—OCH_3$, or $R_1$ together with $R_2$ is $—OCH_2O—$; both $R_3$ and $R_4$ are $—OCH_3$, or $R_3$ together with $R_4$ is $—OCH_2O—$;
$R_5$ is ◄ H, ... $CH_3$ or ◄ OH; and
$R_6$, $R_7$ and $R_8$ are ◄ H or ... $CH_3$;
for the preparation of a therapeutic drug for the treatment of hepatic failure.

2. Use according to claim 1, wherein $R_1$, $R_2$, $R_3$ and $R_4$ are $—OCH_3$, $R_5$ is ◄ H, $R_6$ is ... $CH_3$, $R_7$ is ◄ H and $R_8$ is ... $CH_3$.

3. Use according to claim 1, wherein $R_1$, together with $R_2$ is $—OCH_2O—$, $R_3$ and $R_4$ are $—OCH_3$, $R_5$ is ... $CH_3$, $R_6$ is ◄ H, $R_7$ is ... $CH_3$, $R_8$ is ◄ H.

4. Use according to claim 1, wherein $R_1$, $R_2$, $R_3$ and $R_4$ are $—OCH_3$, $R_5$ is ◄ OH, $R_6$ is ... $CH_3$, $R_7$ is ◄ H and $R_8$ is ... $CH_3$.

5. Use according to claim 1, $R_1$ together with $R_2$ is $—OCH_2O—$, $R_3$ and $R_4$ are $—OCH_3$, $R_5$ is ◄ OH, $R_6$ is ... $CH_3$, $R_7$ is ◄ H, $R_8$ is ... $CH_3$.

6. Use according to claim 1, $R_1$ together with $R_2$ is $—OCH_2O—$, $R_3$ and $R_4$ are $—OCH_3$, $R_5$ is ◄ H, $R_6$ is . . . $CH_3$, $R_7$ is ◄ H and $R_8$ is . . . $CH_3$.

7. Use according to claim 1, wherein $R_1$ together with $R_2$ is $—OCH_2O—$, $R_3$ together with $R_4$ is $—OCH_2O—$, $R_5$ is . . . $CH_3$, $R_6$ is ◄ H, $R_7$ is . . . CH and $R_8$ is ◄ H.

**Patentansprüche**

1. Verwendung einer Verbindung der folgenden allgemeinen Strukturformel:

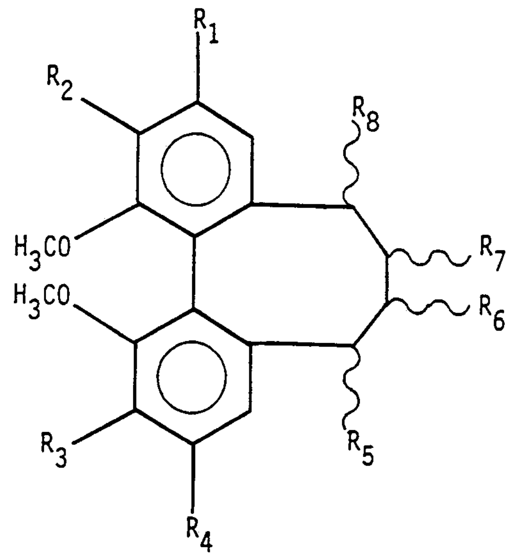

wobei sowohl $R_1$ wie $R_2$ $—OCH_3$ sind oder $R_1$ zusammen mit $R_2$ $—OCH_2O—$ ist;
sowohl $R_3$ wie $R_4$ $—OCH_3$ sind oder $R_3$ zusammen mit $R_4$ $—OCH_2O—$ ist;
$R_5$ ◄ H . . . $CH_3$ oder ◄ OH ist; und
$R_6$, $R_7$ und $R_8$ ◄ H oder . . . $CH_3$ sind;
für die Herstellung eines Arzneimittels für die Behandlung von Leberleiden.

2. Verwendung nach Anspruch 1, wobei $R_1$, $R_2$, $R_3$ und $R_4$ $—OCH_3$ sind, $R_5$ ◄ H, $R_6$ . . . $CH_3$, $R_7$ ◄ H und $R_8$ . . . $CH_3$ ist.

3. Verwendung nach Anspruch 1, wobei $R_1$ zusammen mit $R_2$ $—OCH_2O—$ ist, $R_3$ und $R_4$ $—OCH_3$ sind, $R_5$ . . . $CH_3$, $R_6$ ◄ H, $R_7$ . . . $CH_3$ und $R_8$ ◄ H ist.

4. Verwendung nach Anspruch 1, wobei $R_1$, $R_2$, $R_3$ und $R_4$ $—OCH_3$ sind, $R_5$ ◄ OH, $R_6$ . . . $CH_3$, $R_7$ ◄ H und $R_8$ . . . $CH_3$ ist.

5. Verwendung nach Anspruch 1, wobei $R_1$ zusammen mit $R_2$ $—OCH_3$ ist, $R_3$ und $R_4$ $—OCH_3$ sind, $R_5$ ◄ OH, $R_6$ . . . $CH_3$, $R_7$ ◄ H und $R_8$ . . . $CH_3$ ist.

6. Verwendung nach Anspruch 1, wobei $R_1$ zusammen mit $R_2$ $—OCH_2O—$ ist; $R_3$ und $R_4$ $—OCH_3$ sind, $R_5$ ◄ H; $R_6$ . . . $CH_3$, $R_7$ ◄ H und $R_8$ . . . $CH_3$ ist.

7. Verwendung nach Anspruch 1, wobei $R_1$ zusammen mit $R_2$ $—OCH_2O—$, $R_3$ zusammen mit $R_4$ $—OCH_2O—$, $R_5$ . . . $CH_3$, $R_6$ ◄ H, $R_7$ . . . CH und $R_8$ ◄ H ist.

**Revendications**

1. Utilisation d'un composé représenté par la formule structurale suivante:

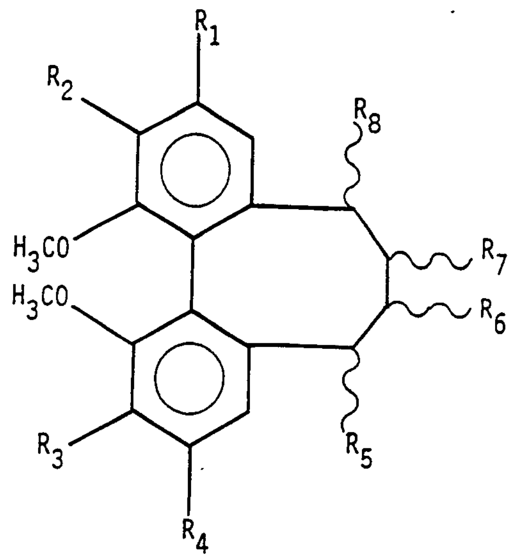

dans laquelle $R_1$ et $R_2$ représentent chacun —$OCH_3$ ou bien $R_1$ ensemble avec $R_2$ représente —$OCH_2O$—; $R_3$ et $R_4$ représentent chacun —$OCH_3$, ou bien $R_3$ conjointement avec $R_4$ représente —$OCH_2O$—; $R_5$ représente ◄ H, . . . $CH_3$ ou ◄ OH; et $R_6$, $R_7$ et $R_8$ représentent ◄ OH ou . . .$CH_3$; pour la préparation d'un médicament thérapeutique pour le traitement de l'insuffisance hépatique.

2. Utilisation selon la revendication 1, dans laquelle $R_1$, $R_2$, $R_3$ et $R_4$ représentent chacun —$OCH_3$, $R_5$ est ◄ H, $R_6$ représente . . . $CH_3$, $R_7$ est ◄ H et $R_8$ est . . . $CH_3$.

3. Utilisation selon la revendication 1, dans laquelle $R_1$ conjointement avec $R_2$ représente —$OCH_2O$—, $R_3$ et $R_4$ représentent chacun —$OCH_3$, $R_5$ représente . . . $CH_3$, $R_6$ représente ◄ H, $R_7$ représente . . . $CH_3$ et $R_8$ représente ◄ H.

4. Utilisation selon la revendication 1, dans laquelle $R_1$, $R_2$, $R_3$ et $R_4$ représentent chacun —$OCH_3$, $R_5$ est ◄ OH, $R_6$ est . . . $CH_3$, $R_7$ est ◄ H et $R_8$ est . . . $CH_3$.

5. Utilisation selon la revendication 1, dans laquelle $R_1$ conjointement avec $R_2$ représente —$OCH_2O$—, $R_3$ et $R_4$ représentent chacun —$OCH_3$, $R_5$ représente ◄ OH, $R_6$ représente . . . $CH_3$, $R_7$ est ◄ H, $R_8$ est . . . $CH_3$.

6. Utilisation selon la revendication 1, dans laquelle $R_1$ conjointement avec $R_2$ représente —$OCH_2O$—, $R_3$ et $R_4$ représentent chacun —$OCH_3$, $R_5$ est ◄ H, $R_6$ est . . . $CH_3$, $R_7$ est ◄ H et $R_8$ est . . . $CH_3$.

7. Utilisation selon la revendication 1, dans laquelle $R_1$ conjointement avec $R_2$ représente —$OCH_2O$—, $R_3$ conjointement avec $R_4$ représente —$OCH_2O$—, $R_5$ est . . . $CH_3$, $R_6$ est ◄ H, $R_7$ représente . . . CH et $R_8$ est ◄ H.